# EUROPÄISCHE PATENTSCHRIFT

(11) **EP 0 539 767 B1**
(45) Veröffentlichungstag und Bekanntmachung des Hinweises auf die Patenterteilung: **21.01.1998**
(21) Anmeldenummer: 92117082.5
(22) Anmeldetag: 07.10.1992
(51) Int. Cl.: C12P 41/00, C12P 13/00

(54) **Enzymatisches Verfahren zur enantioselektiven Herstellung optisch aktiver Cyanhydrine**
Enzymatic process for the enantioselective production of optically active cyanhydrins
Procédé enzymatique pour la production énantiosélective de cyanhydrines optiquement actives

(30) Priorität: 31.10.1991 AT 2174/91
(43) Veröffentlichungstag der Anmeldung: 05.05.1993
(73) Patentinhaber: DSM Chemie Linz GmbH, 4021 Linz (AT)
(72) Erfinder: Griengl, Herfried, Dr., A-8047 Graz (AT); Klempier, Norbert, Dr., A-8044 Graz (AT); Pöchlauer, Peter, Dr., A-4020 Linz (AT)
(74) Vertreter: Kunz, Ekkehard, Dr.

(56) Entgegenhaltungen:
- EP-A- 326 063
- JOURNAL OF THE AMERICAN CHEMICAL SOCIETY, Band 113, 1991 (New York) V.I. OGNYANOV et al. "Pre- paration of Chiral Cyano- hydrins by an Oxynitrilase- - Mediated Transcyanation", Seiten 6992-6996
- CHEMISTRY LETTERS, Nr. 1, 1986 (Tokyo) Y. KOBAYASHI et al. "Asymmetric Transcyanohydri- nation", Seiten 931-934
- Angew.Chemie, Bd.102, Nr.4, 1990, S.423-25

## Beschreibung

Die Erfindung betrifft ein enzymatisches Verfahren zur enantioselektiven Herstellung optisch aktiver Cyanhydrine aus einem Aldehyd oder unsymmetrischen Keton und einem Cyanidgruppendonor unter Einwirkung einer Hydroxynitrillyase.

Cyanhydrine haben etwa für die Gewinnung von alpha - Hydroxysäuren, die zur Gewinnung biologisch wirksamer Stoffe, z.B. pharmazeutischer Wirkstoffe, Vitamine oder auch pyrethroider Verbindungen Verwendung finden, Bedeutung.

Die Herstellung eines Cyanhydrins kann beispielsweise durch Anlagerung einer Cyanidgruppe an den Carbonylkohlenstoff eines Aldehyds oder Ketons erfolgen, wobei bei Einsatz eines Aldehyds oder eines unsymmetrischen Ketons Enantiomerengemische optisch aktiver Cyanhydrine entstehen. Da in einem biologisch wirksamen Enantiomerengemisch im allgemeinen nur eines der beiden Enantiomere biologisch wirksam ist, hat es nicht an Versuchen gefehlt, ein Verfahren zu finden, durch das ein gewünschtes Enantiomeres eines optisch aktiven Cyanhydrins in möglichst hoher optischer Reinheit herstellbar ist.

So ist in Chemistry Letters, The Chemical Society of Japan (1986), 931-934 ein Verfahren zur Cyanhydrinierung eines Aldehyds unter Verwendung eines Cyanidgruppendonors und eines synthetischen Dipeptides als Katalysator geoffenbart. Diese Reaktion verläuft aber nur in geringem Ausmaß enantioselektiv und die optische Reinheit der Produkte ist unbefriedigend.

Aus EP-A-0 326 063 ist bekannt, aliphatische, aromatische oder heteroaromatische Aldehyde oder Ketone mit Blausäure in Gegenwart einer Oxynitrilase umzusetzen, wobei entsprechende R- bzw. S-Cyanhydrine enantioselektiv gebildet werden. Die Handhabung von Blausäure bereitet aber wegen ihres niedrigen Siedepunktes Schwierigkeiten. Außerdem ist Blausäure hochgiftig und wird daher nur sehr ungern in einem technischen Verfahren eingesetzt.

In J.Am.Chem.Soc. 1991, 113, 6992-6996 ist ein Verfahren zur Herstellung von R-Cyanhydrinen durch Umsetzung von aromatischen oder aliphatischen Aldehyden mit Acetoncyanhydrin durch Umsetzung von aromatischen oder aliphatischen Aldehyden mit Acetoncyanhydrin in Gegenwart einer D-Oxynitrilase beschrieben. Um enantiomerenangereicherte Produkte zu erhalten, muß das Verfahren dabei in Gegenwart eines organischen, mit Wasser nicht mischbaren Lösungsmittels durchgeführt werden, da es in wäßriger Lösung allein zu einer Racemisierung des Produktes kommt.

Es wurde nun unerwarteterweise ein enantioselektives Verfahren zur Herstellung des S-Enantiomeren eines optisch aktiven Cyanhydrins gefunden, bei dem die Produkte in hoher optischer Reinheit anfallen und bei dem keine Blausäure und kein organisches Lösungsmittel verwendet werden müssen. S-Cyanhydrine, die von aliphatischen Aldehyden abgeleitet sind, können auf diese Weise erstmals mit Hilfe einer S-Hydroxynitrillyase hergestellt werden.

Gegenstand der Erfindung ist demnach ein enantioselektives Verfahren zur Herstellung des S-Enantiomeren eines optisch aktiven Cyanhydrins durch Umsetzung eines Aldehyds oder eines unsymmetrischen Ketons mit Acetoncyanhydrin, das dadurch gekennzeichnet ist, daß der Aldehyd oder das Keton in einem Verdünnungsmittel in Gegenwart einer S-Hydroxynitrillyase mit dem Acetoncyanhydrin umgesetzt wird, worauf das gebildete S-Cyanhydrin aus der Reaktionsmischung isoliert wird.

Als Ausgangsmaterialien im erfindungsgemäßen Verfahren werden ein Aldehyd oder ein unsymmetrisches Keton, ein Cyanidgruppendonor, eine Hydroxynitrillyase und ein Verdünnungsmittel eingesetzt.

Unter Aldehyden sind dabei aliphatische, aromatische oder heteroaromatische Aldehyde zu verstehen. Unsymmetrische Ketone sind aliphatische, aromatische oder heteroaromatische Ketone, bei denen das Carbonylkohlenstoffatom ungleich substituiert ist. Bevorzugt werden Aldehyde, ganz bevorzugt aliphatische oder aromatische Aldehyde umbesetzt, solche Aldehyde und Ketone sind bekannt oder wie üblich herstellbar.

Als Cyanidgruppendonor wird das Cyanhydrin Acetoncyanhydrin, welches käuflich zu erwerben ist, verwendet.

Als Hydroxynitrillyase kommen S-Hydroxynitrillyasen, z.B. aus *Hevea brasiliensis,* welche sich als besonders geeignet herausgestellt hat, in Frage. Die Hydroxynitrillyase kann dabei gereinigt oder ungereinigt, als solche oder immobilisiert eingesetzt werden. Die Bereitstellung und Reinigung der Hydroxynitrillyase kann beispielsweise durch Fällung mit Ammoniumsulfat und anschließender Gelfiltration, etwa gemäß D.Selmar et al., Physiologia Plantarum 75 (1989), 97-101 erfolgen.

Die Umsetzung erfolgt in einem Verdünnungsmittel. Als besonders vorteilhaft hat es sich herausgestellt, daß die Umsetzung in einem wäßrigen Verdünnungsmittel ohne Zusatz organischer Lösungsmittel, die die Aktivität des Enzyms rasch hemmen, erfolgen kann, wobei es unerwarteterweise zu keiner Racemisierung des Produktes kommt. Die erfindungsgemäße Umsetzung kann aber auch in einem organischen Verdünnungsmittel oder in Gegenwart eines organischen Lösungsmittels ausgeführt werden. Das organische Lösungsmittel kann dabei als Colösungsmittel in einem wäßrigen System oder als Lösungsmittel in einem Zweiphasensystem, beispielsweise in einem Membranreaktor, dienen. Als organische Verdünnungsmittel können aliphatische oder aromatische Kohlenwasserstoffe, die gegebenenfalls halogeniert sind, Alkohole, Ether, Ester verwendet werden. Als organisches Colösungsmittel können mit Wasser mischbare organische Lösungsmittel, etwa Alkohole, als Lösungsmittel in einem Zweiphasensystem mit Wasser nicht mischbare organische Lösungsmittel wie z.B. aliphatische oder aromatische Kohlenwasserstoffe, die gegebenenfalls halogeniert sind, Ether, Ester eingesetzt werden. Bevorzugt erfolgt die Umsetzung nicht in Anwesenheit eines organische Lösungsmittels sondern in einem wäßrigen Verdünnungsmittel. Als wäßriges Verdünnungsmittel wird Wasser, eine wäßrige Salz- oder eine wäßrige Pufferlösung eingesetzt. Bevorzugt wird eine wäßrige Pufferlösung, ganz besonders bevorzugt eine solche, die Natriumcitrat enthält, verwendet. Der pH-Wert soll dabei unter 7, bevorzugt etwa 3 bis 5 betragen.

Pro g Aldehyd oder unsymmetrisches Keton werden etwa 150 bis 300 g Verdünnungsmittel und 500 bis 2000 1U Aktivität Hydroxynitrillyase, bevorzugt etwa 800 bis 1500 1U, zugesetzt. Ein 1U (International Unit) drückt dabei die Bildung von einem Mikromol Produkt pro Minute und pro Gramm Enzymrohisolierung aus. Die benötigte Menge der jeweiligen Hydroxynitrillyase ermittelt man am besten in einem Aktivitätstest, etwa gemäß Selmar et al., Analytical Biochemistry 166 (1987), 208-211.

Pro Mol eingesetzte Aldehyd- oder Ketogruppe werden mindestens ein Mol, bevorzugt 1 bis 2 Mole Acetoncyanhydrin zugegeben.

Die Reaktionsmischung wird bei Temperaturen von etwa 0°C bis zur Desaktivierungstemperatur der Hydroxynitrillyase, bevorzugt von 20 bis 30°C geschüttelt oder gerührt. Dabei wird die Cyanidgruppe vom Acetoncyanhydrin auf das Carbonylkohlenstoffatom des eingesetzten Aldehyds oder Ketons übertragen und es entsteht überwiegend das S-Enantiomere des, dem eingesetzten Aldehyd oder Keton entsprechenden, optisch aktiven Cyanhydrins. Der Fortschritt der Reaktion wird dabei gaschromatographisch verfolgt.

Nach erfolgter Umsetzung kann das gebildete Cyanhydrin aus der Reaktionsmischung mit Hilfe eines organischen Lösungsmittels, das mit Wasser nicht mischbar ist, etwa aliphatische oder aromatische gegebenenfalls halogenierte Kohlenwasserstoffe, z.B. Pentan, Hexan, Benzol, Toluol, Methylenchlorid, Chloroform, Chlorbenzole, Ether wie etwa Diethylether, Diisopropylether oder Ester, beispielsweise Essigsäureethylester oder Mischungen solcher Lösungsmittel extrahiert werden. Sollte die Reinheit des extrahierten Produktes nicht ausreichend sein, kann eine Reinigungsoperation angeschlossen werden. Die Reinigung kann durch eine bekannte Methode erfolgen und gelingt am besten chromatographisch.

In einer bevorzugten Ausführungsform werden etwa 100 mg Aldehyd in 15 bis 30 g einer wäßrigen Pufferlösung mit einem pH-Wert von etwa 4, die Natriumcitrat enthält, mit 2 Mol Acetoncyanhydrin pro Mol eingesetzter Aldehyd- oder Ketogruppe und 200 1U Aktivität Hydroxynitrillyase aus *Hevea brasiliensis* bei Raumtemperatur geschüttelt. Der Fortschritt der Reaktion wird gaschromatographisch verfolgt. Nach beendeter Reaktion wird die Reaktionsmischung wird die Reaktionsmischung mit Methylenchlorid extrahiert, die organische Phase getrocknet und abgedampft. Eine weitere Reinigung des Rückstandes kann säulenchromatographisch erfolgen.

Nach dem erfindungsgemäßen Verfahren werden auf einfache Weise optisch aktive, S-angereicherte Cyanhydrine ohne Blausäure und ohne ein organisches Lösungsmittel verwenden zu müssen, hergestellt. Das Verfahren stellt somit eine Bereicherung der Technik dar.

### Beispiel 1

100 mg Capronaldehyd (1 mMol) wurden in 20 ml o,1 molarem Citratpuffer mit einem pH-Wert von 4 gelöst, mit 1 g Enzymrohisolierung mit einer Aktivität von 100 IU pro Gramm als gefriergetrocknetes Pulver, erhalten gemäß D.Selmar et al., Physiologica Plantarum 75 (1989), 97 bis 101, und 168 mg Acetoncyanhydrin (2 mMol) versetzt und 2 Stunden bei Raumtemperatur am Schüttler geschüttelt. Die Reaktion wurde gaschromatographisch verfolgt. Nach beendeter Reaktion wurde dreimal mit je 25 ml Methylenchlorid extrahiert. Die organischen Phasen wurden vereinigt, über Natriumsulfat getrocknet und das Lösungsmittel am Rotovapor abgedampft.

Dabei wurden 114 mg, das sind 90 % der Theorie, S-Capronaldehydcyanhydrin mit einer Enantiomerenreinheit ee von 84 % erhalten.

### Beispiel 2

80 mg Benzaldehyd (0,75 mMol) und 128 mg Acetoncyanhydrin (1,5 mMol) wurden, wie im Beispiel 1 beschrieben, in 15 ml 0,1 molarem Citratpuffer (pH-Wert = 4) mit 1 g der im Beispiel 1 beschriebenen Enzymrohisolierung umgesetzt.

Dabei wurden 45 mg, das sind 45 % der Theorie, S-Benzaldehydcyanhydrin mit einer Enantiomerenreinheit ee von 94 % erhalten.

Die Bestimmung der optischen Reinheit der gebildeten Aldehydcyanhydrine erfolgte gaschromatographisch auf einer Kapillarsäule als Menthylcarbonat gemäß J.W. Westley et al., J. Org. Chem. 33 (1968), 3978-3980.

Die Bestimmung der optischen Reinheit der Ketoncyanhydrine erfolgte gaschromatographisch auf einer chiralen Trennphase gemäß V. Schurig et al., Ang. Chemie 102 (1990), 969-986.

## Patentansprüche

1. Enantioselektives Verfahren zur Herstellung des S-Enantiomeren eines optisch aktiven Cyanhydrins durch Umsetzung eines Aldehyds oder eines unsymmetrischen Ketons mit Acetoncyanhydrin, dadurch gekennzeichnet, daß der Aldehyd oder das Keton in einem wäßrigen und/oder organischen Verdünnungsmittel in Gegenwart einer S-Hydroxynitril Lyase mit Acetoncyanhydrin umgesetzt wird, worauf das gebildete S-Cyanhydrin aus der Reaktionsmischung isoliert wird, wobei die verwendete S-Hydroxynitril Lyase keine S-Hydroxynitril Lyase aus *Sorghum bicolor* ist.

2. Verfahren nach Anspruch 1, dadurch gekennzeichnet, daß ein aliphatischer, aromatischer oder heteroaromatischer Aldehyd umgesetzt wird.

3. Verfahren nach Anspruch 2, dadurch gekennzeichnet, daß ein aliphatischer oder aromatischer Aldehyd umgesetzt wird.

4. Verfahren nach einem der Ansprüche 1 bis 3, dadurch gekennzeichnet, daß als Verdünnungsmittel ein wäßriges Verdünnungsmittel eingesetzt wird.

5. Verfahren nach Anspruch 4, dadurch gekennzeichnet, daß die Umsetzung in einer wäßrigen Pufferlösung bei einem pH-Wert von 3 bis 5 durchgeführt wird.

6. Verfahren nach einem der Ansprüche 1 bis 5, dadurch gekennzeichnet, daß als Hydroxynitril Lyase eine S-Hydroxynitril Lyase aus *Hevea brasiliensis* eingesetzt wird.

7. Verfahren nach einem der Ansprüche 1 bis 6, dadurch gekennzeichnet, daß das gebildete S-Cyanhydrin aus der Reaktionsmischung extrahiert und chromatographisch gereinigt wird.

8. Verwendung einer S-Hydroxynitril Lyase aus *Hevea brasiliensis* zur Herstellung von S-Cyanhydrinen.

## Claims

1. Enantioselective process for the preparation of the *S* enantiomer of an optically active cyanohydrin by reacting an aldehyde or an asymmetric ketone with acetone cyanohydrin, characterized in that the aldehyde or the ketone is reacted with acetone cyanohydrin in an aqueous and/or organic diluent in the presence of an *S*-hydroxynitrile lyase, whereupon the *S*-cyanohydrin formed is isolated from the reaction mixture, the *S*-hydroxynitrile lyase used not being an *S*-hydroxynitrile lyase from *Sorghum bicolor.*

2. Process according to Claim 1, characterized in that an aliphatic, aromatic or heteroaromatic aldehyde is reacted.

3. Process according to Claim 2, characterized in that an aliphatic or aromatic aldehyde is reacted.

4. Process according to one of Claims 1 to 3, characterized in that an aqueous diluent is employed as diluent.

5. Process according to Claim 4, characterized in that the reaction is carried out in an aqueous buffer solution at a pH of 3 to 5.

6. Process according to one of Claims 1 to 5, characterized in that an *S*-hydroxynitrile lyase from *Hevea brasiliensis* is employed as hydroxynitrile lyase.

7. Process according to one of Claims 1 to 6, characterized in that the *S*-cyanohydrin formed is extracted from the reaction mixture and purified by chromatography.

8. Use of an S-hydroxynitrile lyase from *Hevea brasiliensis* for the preparation of *S*-cyanohydrins.

## Revendications

1. Procédé énantiosélectif pour la préparation de l'énantiomère S d'une cyanhydrine optiquement active par réaction d'un aldéhyde ou d'une cétone asymétrique avec de l'acétonecyanhydrine, caractérisé en ce qu'on fait réagir l'aldéhyde ou la cétone dans un diluant aqueux et/ou organique en présence d'une S-hydroxynitrilase, avec l'acétone-cyanhydrine, après quoi on isole du mélange de réaction la S-cyanhydrine formée, procédé dans lequel la S-hydroxynitrilase qu'on utilise n'est pas une S-hydroxynitrilase de *Sorghum bicolor.*

2. Procédé selon la revendication 1, caractérisé en ce qu'on fait réagir un aldéhyde aliphatique, aromatique ou hétéroaromatique.

3. Procédé selon la revendication 2, caractérisé en ce qu'on fait réagir un aldéhyde aliphatique ou aromatique.

4. Procédé selon l'une des revendications 1 à 3, caractérisé en ce qu'on utilise un diluant aqueux comme diluant.

5. Procédé selon la revendication 4, caractérisé en ce qu'on effectue la réaction dans une solution tamponnée aqueuse à pH 3 à 5.

6. Procédé selon l'une des revendications 1 à 5, caractérisé en ce qu'on utilise, comme hydroxynitrilase, une S-hydroxynitrilase de *Hevea brasiliensis.*

7. Procédé selon l'une des revendications 1 à 6, caractérisé en ce qu'on extrait la S-cyanhydrine formée du mélange de réaction et on la purifie par chromatographie.

8. Utilisation d'une S-hydroxynitrilase de *Hevea brasiliensis* pour la préparation de S-cyanhydrines.
